# EUROPÄISCHE PATENTSCHRIFT

(11) **EP 1 447 140 B1**
(45) Veröffentlichungstag und Bekanntmachung des Hinweises auf die Patenterteilung: **30.07.2008**
(21) Anmeldenummer: 04001419.3
(22) Anmeldetag: 23.01.2004
(51) Int. Cl.: B05B 11/02

(54) **Austragvorrichtung zur manuellen Erzeugung eines Volumenstroms**
Discharging device for manually producing a given volume flow
Dispositif de décharge pour produire manuellement un écoulement d'un volume donné

(30) Priorität: 12.02.2003 DE 10306686
(43) Veröffentlichungstag der Anmeldung: 18.08.2004
(73) Patentinhaber: ING. ERICH PFEIFFER GMBH, 78315 Radolfzell (DE)
(72) Erfinder: Greiner-Perth, Jürgen, 78244 Gottmadingen (DE)
(74) Vertreter: Patentanwälte Ruff, Wilhelm, Beier, Dauster & Partner

(56) Entgegenhaltungen:
- EP-A- 0 546 607
- FR-A- 2 761 281

## Beschreibung

Die Erfindung betrifft eine Austragvorrichtung zur manuellen Erzeugung eines Volumenstroms durch einen Austraghub, mit einem Mediumspeicher für zumindest ein Medium, einer Austragöffnung, einer Pumpeinrichtung und einem der Pumpeinrichtung zugeordneten Arbeitsbereich, der in einer Ruheposition über eine Rasteinrichtung eine lösbare, formschlüssige Verbindung zu einem Haltebereich aufweist.

Derartige Austragvorrichtungen sind aus dem Stand der Technik in vielfältigen Ausführungen bekannt, beispielsweise aus EP 0 546 607. Ein Austrag zumindest eines Mediums, insbesondere eines pulverisierten Feststoffes, einer Flüssigkeit mit wässriger bis zähfließender Konsistenz oder eines Gases ist insbesondere für viele Bereiche der Kosmetik und Pharmazie von großer Bedeutung. Dazu wird das zumindest eine Medium in einem Mediumspeicher der Austragvorrichtung gelagert und kann mittels einer manuell zu betätigenden Pumpeinrichtung durch eine Austragöffnung hindurch in die Umgebung befördert werden. Die Pumpeinrichtung setzt insbesondere das Medium im Mediumspeicher unter Druck und bewirkt damit den Austrag des Mediums durch die Austragöffnung. Die Austragöffnung ist dabei auf die Viskosität und den Anwendungsbereich des auszutragenden Mediums abgestimmt. Insbesondere für ein Medium mit geringer Partikelgröße, wässriger Konsistenz und niedriger Viskosität wird häufig eine Zerstäubung in der Umgebungsluft mittels einer düsenförmigen Austragöffnung angestrebt. Für ein zähflüssiges, hochviskoses Medium wird dagegen insbesondere eine zylindrische oder konische Austragöffnung bevorzugt.

Für den Austrag des Mediums ist ein Arbeitsbereich vorgesehen, der in einer Ruheposition über eine Rasteinrichtung eine lösbare, formschlüssige Verbindung zu einem Haltebereich aufweist und somit eine ungewollte Betätigung der Pumpeinrichtung verhindert. Um den Austrag des Mediums zu bewirken, wird vom Benutzer eine Betätigungskraft auf die Austragvorrichtung ausgeübt, die zu einer Relativbewegung zwischen dem Haltebereich und dem Arbeitsbereich führt. Diese Relativbewegung wird allerdings erst möglich, wenn die vom Benutzer auf die Austragvorrichtung ausgeübte Betätigungskraft eine durch die formschlüssige Verbindung zwischen Arbeitsbereich und Haltebereich bestimmte Bedienkraft überschreitet. Dadurch wird die formschlüssige Verbindung zwischen dem Arbeitsbereich und dem Haltebereich gelöst. Anschließend wird insbesondere der gewünschte Druckaufbau im Mediumspeicher der Austragvorrichtung bewirkt.

Austrageinrichtungen, die für eine geringe Anzahl von Pumphüben, insbesondere für ein oder zwei Pumphübe ausgelegt sind, weisen häufig einen versiegelten Mediumspeicher auf. Dazu wird die Pumpeinrichtung so ausgelegt, dass das auszutragende Medium erst unmittelbar bei der Durchführung des Pumpenhubes aus dem bis zu diesem Zeitpunkt versiegelten Mediumspeicher herausbefördert wird. Die Pumpeinrichtung öffnet beim ersten Pumpenhub die Versiegelung des Mediumspeichers und setzt diesen unter Druck. Derartige Austragvorrichtungen, die für eine geringe Anzahl von Pumphüben ausgelegt sind, werden insbesondere für die Verabreichung von Impfstoffen und/oder anderen hochwirksamen Arzneistoffen benutzt. Da hierbei insbesondere nur ein ein- oder zweimaliges Betätigen der Pumpeinrichtung notwendig ist und die Austragvorrichtung aus hygienischen Gründen danach zu entsorgen ist, besteht ein großes Interesse an besonders preisgünstigen und dennoch zuverlässigen Austragvorrichtungen.

Bei der Verwendung derartiger Austragvorrichtungen für Impfstoffe, die insbesondere für die Verwendung bei Epedemien oder Katastrophenfällen vorgesehen sind und daher über einen langen Zeitraum eingelagerungsfähig sein müssen, müssen Alterungseffekte berücksichtigt werden. Eine Alterung findet nicht nur hinsichtlich des in der Austragvorrichtung gespeicherten Mediums, sondern auch bezüglich der Austragvorrichtung als solcher statt.

Austragvorrichtungen werden insbesondere aus Kunststoffmaterialien hergestellt, bei denen selbst bei optimalen Lagerungsbedingungen über einen längeren Zeitraum eine Veränderung der Werkstoffeigenschaften wie Materialdichte, Materialvolumen, Elastizitätsmodul und Kerbschlagzähigkeit auftritt. Durch eine derartige Alterung der Kunststoffmaterialien kann es zu einer Veränderung der Bedienkräfte oder zu einem Versagen der Austragvorrichtung im Anwendungsfall kommen, wodurch die Anwendung des gespeicherten Mediums in Frage gestellt wird.

Aufgabe der Erfindung ist es, eine Austragvorrichtung der eingangs genannten Art zu schaffen, die die Wirkung der Alterungseffekte der verwendeten Materialien auf die Austrageinrichtung minimiert.

Diese Aufgabe wird dadurch gelöst, dass die Rasteinrichtung in zumindest einer Ebene orthogonal zu einer Austraghubrichtung einen zumindest abschnittsweise oder umlaufend gewellten und/oder polygonförmigen Querschnitt aufweist. Die Austraghubrichtung bestimmt sich durch die Gestaltung des Arbeitsbereiches und des Haltebereiches, die insbesondere so aufeinander abgestimmt werden, dass lediglich ein translatorischer Freiheitsgrad der Bewegung für die Pumpeinrichtung ermöglicht wird. In einer Ebene orthogonal zur Austraghubrichtung ist die Rasteinrichtung vorzugsweise so gestaltet, dass sie einen zumindest abschnittsweise oder, besonders bevorzugt, umlaufend gewellten Querschnitt aufweist. Dieser Querschnitt kann insbesondere durch eine stetige oder unstetige Aneinanderreihung mehrerer Kreisbogensegmente, Geradenstücke oder Kombinationen davon geschaffen werden. Durch die gerundeten Übergänge einer derartigen Gestaltung der Rasteinrichtung ergeben sich zusätzliche herstellungs- und festigkeitsmäßige Vorteile gegenüber einem zumindest abschnittsweise polygonförmigen Querschnitt. In einer bevorzugten Ausführungsform umschließt die Rasteinrichtung den zylindrisch ausgeführten Arbeitsbereich insbesondere kreisförmig, wobei durch den gewellten Querschnitt eine großflächige Anlage zwischen Rasteinrichtung und Arbeitsbereich vermieden werden kann. Vielmehr treten Arbeitsbereich und Rasteinrichtung nur punktförmig und/oder linienförmig und/oder flächig miteinander in Kontakt. Dies führt bei Betätigung der Austragvorrichtung zu einem gut vorherbestimmbaren Reibungswiderstand zwischen Arbeitsbereich und Rasteinrichtung. Der Reibungswiderstand erfährt durch Alterungseffekte keine signifikante Veränderung, da durch den gewellten Querschnitt eine Begrenzung einer die Reibkraft bestimmenden Normalkraft erreicht werden kann. Daher kann bei einer derartigen Gestaltung der Rasteinrichtung in Verbindung mit dem Arbeitsbereich eine langfristige Funktionsfähigkeit der Austragvorrichtung gewährleistet werden.

In Ausgestaltung der Erfindung ist an mindestens einem, dem Arbeitsbereich zugewandten Wellvorsprung der Rasteinrichtung ein Rastelement vorgesehen. Ein Wellvorsprung ist ein zumindest ein durch den abschnittsweise oder umlaufend gewellten Querschnitt ausgeformter, insbesondere konvexer Bereich der Rasteinrichtung. Der Wellvorsprung ist dem Arbeitsbereich zugewandt und weist gegenüber anderen Bereichen der Rasteinrichtung einen minimalen Abstand zum Arbeitsbereich auf. Ein Rastelement stellt eine formschlüssige und/oder kraftschlüssige Verbindung zwischen der Rasteinrichtung und dem Arbeitsbereich her und ist insbesondere als erhabener Bereich, Rast- oder Schnappnase ausgeführt. Am Arbeitsbereich ist zumindest eine Ein- oder Ausformung, insbesondere eine Tasche, eine Nut, ein Bund oder ein Steg vorgesehen. Mit dieser Ein- oder Ausformung tritt das Rastelemelement formschlüssig, insbesondere durch Hintergreifen, in Verbindung. Eine Anordnung des Rastelementes auf einem dem Arbeitsbereich zugewandten Wellvorsprung ermöglicht die Gestaltung des Rastelementes als kompakte und dadurch stark belastbare Geometrie, auf die auch während des Austraghubes nur geringe Kräfte einwirken. Dadurch kann die Funktion des Rastelementes auch bei Alterung und Versprödung des Werkstoffes gewährleistet werden.

In weiterer Ausgestaltung der Erfindung ist die Rasteinrichtung in dem zumindest abschnittsweise oder umlaufend gewellten Querschnittsbereich durch eine einheitliche oder abschnittsweise variierende Wandstärke zumindest abschnittsweise flexibel gestaltet. Eine Flexibilität des Rastbereiches ist für einen Ausrastvorgang aus dem formschlüssigen Hintergreifen mit dem Arbeitsbereich erforderlich. Dabei wird die Rasteinrichtung elastisch verformt, so dass ein Ausgleiten des oder der Rastelemente aus der Ein- oder Ausformung des Arbeitsbereiches erfolgen kann. Die dazu notwendige Flexibilität kann entweder durch das Rastelement als solches oder durch den Wellvorsprung, auf dem das Rastelement angebracht ist, bewirkt werden. Durch den gewellten Querschnitt wird eine Elastizität der Rasteinrichtung hervorgerufen, die einerseits den Anforderungen der Austragvorrichtung während des Austraghubes entspricht und andererseits auch bei einer Alterung des Werktstoffes ein Versagen der Rasteinrichtung nahezu ausschließt. Dies wird insbesondere dadurch bewirkt, dass im Gegensatz zum Stand der Technik, wo insbesondere aus umgebenden Material freigestellte, federnde Zungen für Rastelemente verwendet werden, eine homogene Spannungsverteilung in der Rasteinrichtung gewährleistet ist. Weiterhin sind durch die erfindungsgemäße Gestaltung der Rasteinrichtung keine gekerbten oder eingeschnittenen Bereiche notwendig, in denen Spannungsspitzen auftreten könnten und die daher für die Ausbildung von Rissen und einem darauf nachfolgenden Versagen prädestiniert wären.

In weiterer Ausgestaltung der Erfindung weist das Rastelement an einer dem Wellvorsprung abgewandten Stirnfläche eine Kontur auf, die der Geometrie der Arbeitsbereiches angepasst ist und die als Führungselement für den Arbeitsbereich vorgesehen ist. Eine dem Wellvorsprung abgewandte Stirnfläche des Rastelementes weist mit ihrer Flächennormale zumindest nahezu in Richtung des Arbeitsbereiches und tritt als Kontaktfläche mechanisch mit einer Oberfläche des Arbeitsbereiches in Verbindung. Da es während eines Austraghubes der Austragvorrichtung zu einer Relativbewegung zwischen der mit dem Haltebereich verbundenen Rasteinrichtung und dem Arbeitsbereich kommt, ist ein reibungsund verschleissarmer Gleitvorgang zwischen dem Arbeitsbereich und dem zumindest einen auf der Oberfläche des Arbeitsbereiches gleitenden Rastelement wünschenwert. Um hierfür gute Gleiteigenschaften zu bewirken, ist die dem Arbeitsbereich zugewandte Stirnfläche des Rastelementes auf die Geometrie des Arbeitsbereiches angepasst und dient als Führungselement für den Arbeitsbereich. Eine derartige Anpassung kann insbesondere durch die Gestaltung der Stirnfläche des Rastelementes als konkave oder konvexe Fläche, als Kugelabschnitt, als Prismenkante oder als Kegel- oder Pyramidenspitze erfolgen. Das Rastelement bewirkt weiterhin durch die Anpassung der Stirnfläche auf die Oberfläche des Arbeitsbereiches eine Gleitführung des Arbeitsbereiches.

Damit verbunden ist die Beschränkung der Bewegungsfreiheit des Arbeitsbereiches auf eine translatorische Bewegung.

In weiterer Ausgestaltung der Erfindung ist der Arbeitsbereich an einer Außenfläche des Mediumspeichers vorgesehen. Dadurch lässt sich eine besonders einfache Aufbauweise der Austragvorrichtung erreichen, wie sie insbesondere bei Austragvorrichtungen für Einmaldosierungen bevorzugt wird. Dazu wird der Mediumspeicher an seiner Außenfläche mit den für den formschlüssigen Eingriff der Rasteinrichtung notwendigen Aus- oder Einformungen versehen. Bei Aufbringen einer Kraft auf die Austragvorrichtung durch den Benutzer kann bei Überschreiten der durch die Rasteinrichtung vorgegebenen Betätigungskraft die Relativbewegung zwischen Haltebereich und Arbeitsbereich hervorgerufen werden, wodurch der Austrag des Mediums bewirkt wird. Ein zwei- oder mehrfacher Medienaustrag ist mit einer derartigen Anordnung ebenfalls möglich. Um hierfür einen zuverlässigen, schrittweisen Austrag des Mediums zu gewährleisten, sind jedoch zusätzliche Maßnahmen hinsichtlich der Gestaltung der Aus- oder Einformungen an der Außenfläche des Mediumspeichers vorzusehen.

In weiterer Ausgestaltung der Erfindung ist der Arbeitsbereich an einer Außenfläche einer Vorschubhülse vorgesehen, wobei die Vorschubhülse den Mediumspeicher zumindest abschnittsweise umgreift, die Vorschubhülse vorzugsweise einen Ruherastbereich und zumindest einen davon beabstandeten Funktionsrastbereich aufweist und begrenzt verschieblich in dem Haltebereich gelagert ist und/oder von einer Druckhülse ansteuerbar angeordnet ist und zumindest eine lösbare, formschlüssige Druckposition zwischen der Vorschubhülse und der Druckhülse vorgesehen ist. Durch die Verwendung einer Vorschubhülse wird eine Entkopplung des Mediumspeichers von dem Haltebereich erreicht, wodurch sich eine Zwei- oder Mehrfachdosierung der Austragvorrichtung in besonders vorteilhafter Weise verwirklichen lässt. Die Vorschubhülse überträgt während des Austraghubes die vom Benutzer aufgebrachte Kraft auf den Mediumspeicher. Nach dem Austrag der für eine erste Dosierung vorgesehenen Menge des Mediums verhindert die Vorschubhülse ein unmittelbares, weiteres Austragen. Dadurch wird der Benutzer in die Lage versetzt, einen zweiten und/oder weiteren Austraghub mit einer ebenfalls definierten Medienmenge durchzuführen, ohne dass er hierfür besondere Vorkehrungen treffen bzw. besondere Aufmerksamkeit walten lassen muss.

Der Ruherastbereich der Vorschubhülse steht in einem Ausgangszustand der Austragvorrichtung mit den Rastelementen der Rasteinrichtung formschlüssig in Verbindung. Er bewirkt eine Dosierbereitschaft der Austragvorrichtung bei gleichzeitiger Lagerungs- und Transportstabilität.

Durch die Aufbringung einer Betätigungskraft auf die Austragvorrichtung durch den Benutzer wird die formschlüssige Verbindung zwischen dem Ruherastbereich der Vorschubhülse und der Rasteinheit gelöst und eine Relativbewegung entlang des Arbeitsbereiches der Vorschubhülse und dem Haltebereich ermöglicht. Zumindest ein Teil dieser Relativbewegung entspricht dem ersten Austraghub, der insbesondere durch ein Einrasten der Rasteinrichtung in den ersten Funktionsrastbereich beendet werden kann. Nach diesem ersten Austraghub hat der Benutzer die Möglichkeit, die Austragvorrichtung kurz- oder längerfristig ohne eine Kraftbeaufschlagung zu belassen. Zumindest ein weiterer Austraghub kann dann durch Überwindung der Rastkräfte des Funktionsrastbereiches durchgeführt werden. Die Anordnung des Ruherastbereiches und des mindestens einen davon beabstandeten Funktionsrastbereiches entspricht insbesondere dem für einen Mediumaustrag notwendigen Austraghub. Die Druckhülse schließt die Austragvorrichtung auf einer der Austragöffung abgewandten Seite ab. An der Druckhülse ist eine Fingeauflage zur Betätigung der Pumpeinrichtung angebracht. Die Druckhülse kann insbesondere erst im Anwendungsfall der Austragvorrichtung in die formschlüssige Druckposition mit der Vorschubhülse gebracht werden, wodurch eine zusätzliche Sicherung gegen unbeabsichtigte Betätigung erreicht werden kann.

In weiterer Ausgestaltung der Erfindung ist die Druckhülse von einem Startrastbereich in zumindest einen weiteren Druckrastbereich entlang der Vorschubhülse überführbar. Durch die Verwendung der Druckhülse zur Übertragung der vom Benutzer aufgebrachten Kraft auf die Vorschubhülse kann eine mehrfache Betätigung der Austragvorrichtung bei einer gleichbleibenden Außengeometrie der Austragvorrichtung verwirklicht werden. Die Vorschubhülse verschiebt sich dabei mit jedem Austraghub schrittweise in Richtung des Austraghubes. Die Druckhülse gleitet nach einem ersten Austraghub und nachfolgender Reduzierung der Betätigungskraft aus dem Startrastbereich in den zumindest einen Druckrastbereich und stellt eine gleichbleibende Außengeometrie der Austragvorrichtung sicher. Dadurch lassen sich für den Benutzer gleichbleibende Benutzungsbedingungen, insbesondere hinsichtlich der Aufbringung von Betätigungskräften erreichen.

In weiterer Ausgestaltung der Erfindung hat die Rasteinrichtung die Form einer gewellten Hülse, wobei im Bereich einer ersten Stirnfläche der Hülse zumindest ein Rastelement und an einer weiteren Stirnfläche eine vorzugsweise als Halteflansch ausgebildete Versteifung vorgesehen ist. Dadurch kann eine unkontrollierte Aufweitung der Rasteinrichtung während der elastischen Deformation im Anwendungsfall der Austragvorrichtung verhindert werden. Weiterhin wird sichergestellt, dass auch bei fortgeschrittener Alterung der Rasteinrichtung die ursprünglich vorgesehenen Rastkräfte innerhalb eines Toleranzbandes liegen und somit die ordnungsgemäße Funktion der Rasteinrichtung gewährleistet ist.

In weiterer Ausgestaltung der Erfindung sind die Druckhülse, die Vorschubhülse, das Rastelement und der Mediumspeicher über insbesondere formschlüssig wirkende Rasteinrichtungen so aufeinander abgestimmt, dass zumindest ein zweifacher Austraghub der Austragvorrichtung durchführbar ist. Dabei sind die Rasteinrichtungen so angeordnet, dass sie nach der Durchführung eines ersten Austraghubes eine Zwischenstellung für die Vorschubhülse und den Medienspeicher ermöglichen, während die Druckhülse in ihre Ausgangsstelung zurückkehrt, bei der sie jedoch eine neue Rastposition gegenüber der Vorschubhülse einnimmt. Somit kann zumindest ein weiterer Austraghub durch den Benutzer vorgenommen werden, der die Vorschubhülse und den damit gekoppelten Mediumspeicher in eine nächste Zwischenstellung oder eine Endstellung verschiebt.

Die Aufgabe wird auch dadurch erfindungsgemäß gelöst, dass die Rasteinrichtung in zumindest einer Ebene orthogonal zu einer Austraghubrichtung einen zumindest abschnittsweise oder umlaufend gewellten Querschnitt aufweist, wobei eine den Medienspeicher zumindest abschnittsweise umgreifende Vorschubhülse vorgesehen ist, die für eine formschlüssige Rastverbindung mit der Rasteinrichtung und einer Druckhülse jeweils zumindest zwei voneinander beabstandete Rastbereiche aufweist, und die von der Druckhülse derart ansteuerbar vorgesehen ist, dass durch eine von einem Benutzer mehrfach aufgebrachte Betätigungskraft zumindest ein zweimaliger Austrag des Mediums vornehmbar ist.

Weitere Vorteile und Merkmale der Erfindung ergeben sich aus den Ansprüche und der nachfolgenden Beschreibung eines bevorzugten Ausführungsbeispiels der Erfindung, das anhand der Zeichnungen dargestellt ist.
- Fig. 1: zeigt in ebener Schnittansicht eine bevorzugte Ausführung der erfindungsgemäßen Austragvorrichtung in einer Ruheposition,
- Fig.2: in perspektivischer, geschnittener Darstellung eine Rasteinrichtung.

Eine Austragvorrichtung 1 weist ein Grundgehäuse 5 auf, das als rotationssymmetrischer Körper mit einem zylindrischen und am Ende abgerundeten Applikationsteil ausgeführt ist. Weiterhin ist eine Fingerauflage 8 vorgesehen, die an dem Applikationsteil anschließt und die als umlaufender Bund gestaltet ist. Daran anschließend liegt ein im wesentlichen zylindrisch gestalteter Aufnahmeraum 7, der durch eine umlaufende zylindrische Wandung begrenzt wird.

An einer dem Applikationsbereich abgewandten Stirnseite wird der Aufnahmeraum 7 durch eine verschieblich im Aufnahmeraum 7 angebrachte Druckhülse 4 zumindest teilweise abgeschlossen. Die Druckhülse 4 weist an ihrem in Richtung des Applikationsteils gerichteten Ende einen Anschlagbund 20 auf, der einen vergrößerten Außendurchmesser gegenüber der ansonsten im wesentlichen zylindrischen Außenkontur der Druckhülse 4 hat. Der Anschlagbund 20 der Druckhülse 4 ist so dimensioniert, dass er auf einer durch die Innenwandung des Aufnahmeraums 7 gebildeten, zylindrischen Innenfläche längs einer gemeinsamen Mittelachse des Aufnahmeraumes 7 und der Druckhülse 4 gleiten kann. An einem dem Applikationsteil zugewandten Ende der Druckhülse 4 ist ein Zentrierkonus 33 vorgesehen, der bei der Montage der Druckhülse 4 in den Aufnahmeraum 7 an einer dort vorgesehenen Einführschräge 22 abgleitet und somit ein Einschieben der Druckhülse 4 erleichtert. Die Druckhülse 4 wird mittels einer Schraubenfeder 23 in einer Ruheposition gehalten. Sie weist an ihrem dem Applikationsteil abgewandten Ende einen nach innen gerichteten Bund auf, der bei der Betätigung der Austragvorrichtung 1 durch den Benutzer als Daumenauflage 24 benutzt wird. Die Daumenauflage erstreckt sich ab einem bestimmten Innendurchmesser als abschnittsweise geschlitzter Druckkonus 44 in Form einer konisch geformten Wandung in Richtung des Applikationsbereiches.

Der abschnittsweise geschlitzte Druckkonus 44 der Druckhülse 4 umschließt eine im wesentlichen zylindrisch ausgeführte Vorschubhülse 9 und ist mit dieser an einem als umlaufenden Bund ausgeführten Startrastbereich 32 in Richtung eines Austraghubes A formschlüssig verbunden. Der Startrastbereich 32 ermöglicht bei Aufbringung einer Druckkraft auf die Daumenauflage 24 eine Kraftübertragung von der Druckhülse 4 auf die Vorschubhülse 9. Entgegen der längs der Mittelachse der Druckhülse und des Grundgehäuses in Richtung des Applikationsteiles verlaufenden Austraghubrichtung A ist ein vom Startrastbereich 32 beabstandeter Druckrastbereich 31 an der Vorschubhülse 9 vorgesehen. Die Vorschubhülse 9 wird an einem dem Applikationsbereich abgewandten Ende durch den Vorschubhülsenboden 30 verschlossen. Oberhalb des Startrastbereiches 32 sind an einer Außenkontur der Vorschubhülse 9 zwei voneinander beabstandete Rastringe 21 sowie je ein von dem jeweiligen Rastring 21 in unmittelbarer Nähe beabstandeter Haltering 34 vorgesehen. Je ein Rastring 21 und ein Haltering 34 bilden einen Ruherastbereich 36 sowie einen Funktionsrastbereich 37 der Vorschubhülse 9.

Die Vorschubhülse 9 ist im wesentlichen mit einer sich nahezu über die gesamte Länge erstreckten, zylindrischen Bohrung versehen, deren Durchmesser in der Nähe des Startrastbereiches 32 bis kurz vor den Vorschubhülsenboden 30 reduziert ist. Weiterhin ist an einer Innenwandung der Vorschubhülse 9 rotationssymmetrisch zirkular um jeweils 120° versetzt eine Führungsnase 46 vorgesehen. Darüber hinaus ist ein einem dem Applikationsbereich zugewandten Ende der Vorschubhülse 9 ein umlaufender Führungsbund 45 vorgesehen.

Auf dem Führungsbund 45 und den Führungsnasen 46 gleitet bei einem Austraghub ein Mediumspeicher 2, der als im wesentlichen zylindrisches, becherartiges und in Richtung des Applikationsbereiches geöffnetes Behältnis von der Vorschubhülse 9 abschnittsweise umschlossen wird. Der Mediumspeicher 2 ist mit einem Medium 17 gefüllt und mit einem Dichtelement 10 verschlossen. Das Dichtelement 10 ist aus einem Elastomer hergestellt und als rotations- und spiegelsymmetrischer Körper ausgeführt, wobei es an einer Außenkontur mehrere als Dichtringe wirkende Rippen aufweist. Entlang der Symmetrieachse des Dichtelementes 10 ist jeweils eine Sacklochbohrung in und gegen die Richtung der Symmetrieachse vorgesehen.

An einer dem Applikationsbereich zugewandten Kolbendruckfläche 16 wird das Dichtelement 10 während des Austraghubes durch eine Stößeldruckfläche 15 eines Pumpenstößels 3 mit einer Druckkraft beaufschlagt, wodurch das verschieblich im Mediumspeicher 2 angebrachte Dichtelement 10 einen Gegendruck im gespeicherten Medium 17 hervorruft. Der Pumpenstößel 3 ist in einer Stößelaufnahme 12 des Grundgehäuses 5 geführt, er weist eine im wesentlichen zylindrische und rotationssymmetrische Gestalt auf und beherbergt nahe seiner Symmetrieachse einen als Mediumröhre 11 ausgeführten Mediumkanal 6. Der Mediumkanal 6 wird in Richtung des Applikationsteils im Ruhezustand durch einen Ventilblock 38, der in einer Ventilkammer 39 des Grundgehäuses 5 vorgesehen ist, verschlossen. Oberhalb des Ventilblocks 38 an einem Austritt der Ventilkammer 39 in die Umgebung ist eine Austragdüse 40 vorgesehen, die durch einen starken Querschnittssprung zwischen dem Durchmesser der Austragdüse 40 und der Umgebung eine Vernebelung des ausgetragenen Mediums 17 bewirkt.

An einer dem Applikationsbereich abgewandten Stirnseite der Mediumröhre 11 ist diese keilförmig angeschnitten und bildet dadurch eine Schneidspitze 14. Während des Austraghubes gleitet die Schneidspitze 14 in die im Dichtelement 10 vorgesehene Bohrung und durchtrennt dort das Dichtelement 10. Dadurch kann die Mediumröhre 11 in das im Mediumspeicher 2 gelagerte Medium 17 eintauchen.

Um eine ungewollte Betätigung der Austragvorrichtung 1 zu verhindern, sind zwischer einer als Arbeitsbereich wirkenden Außenkontur 35 der Vorschubhülse 9 und einem Wellelement 25 drei zirkular um die Symmetrieachse der Austragvorrichtung im Winkel von jeweils 120° angebrachte Rastelemente 19 vorgesehen, die in den Ruherastbereich 36 der Vorschubhülse 9 formschlüssig eingreifen. Weiterhin ist eine als Druckfeder wirkende Schraubenfeder 23 in dem Aufnahmeraum 7 sowie in einem von der Druckhülse gebildeten Arbeitsraum vorgesehen, die die Druckhülse 4 formschlüssig gegen eine von der Außenwand des Aufnahmeraums 7 gebildete, umlaufende Anschlagkante 18 presst.

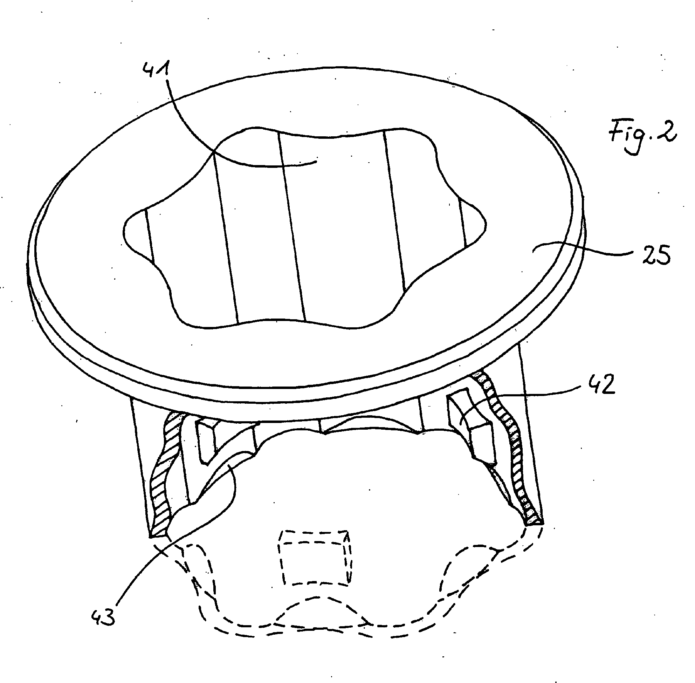

Wie in Fig. 2 dargestellt, ist das Wellelement 25 aus einer Scheibe aufgebaut, die durch eine aus mehreren stetig aneinandergereihten Kreisbogensegmenten gebildeten Kontur durchbrochen wird: Diese Kontur erstreckt sich über die Dicke der Scheibe in Richtung einer Flächennormale der Scheibe und wird von einer dünnen Wandung berandet. An dieser Wandung sind bei der vorliegenden Ausführungsform an drei von insgesamt sechs Wellvorsprüngen 41 jeweils auf dem Scheitel des konvex nach innen gerichteten Wellvorsprungs 41 Rastelemente 42 vorgesehen, die an einer dem Wellvorsprung 41 abgewandten Stirnseite auf die Kontur des durch die Vorschubhülse 9 gebildeten Arbeitsbereiches 35 angepasst sind.

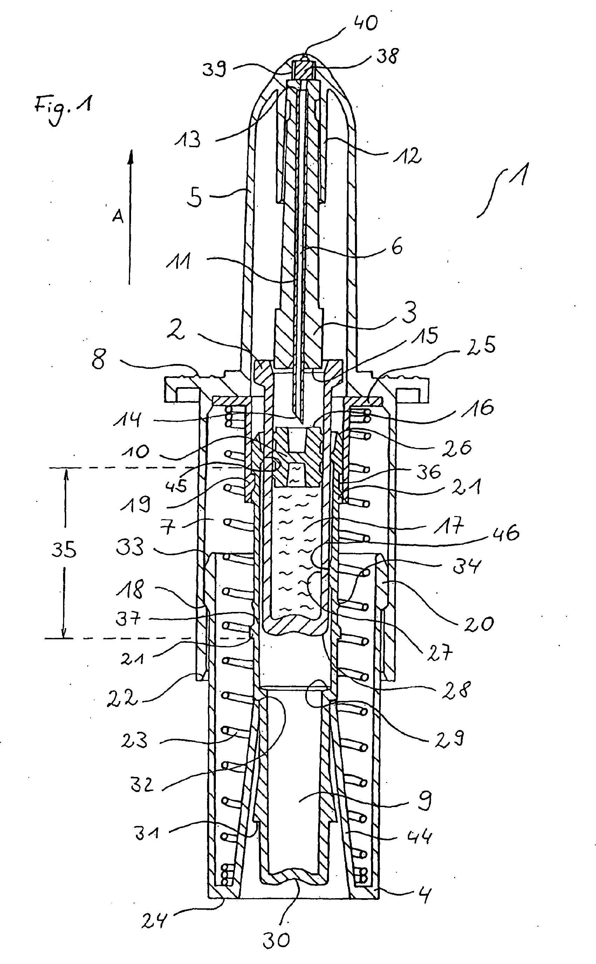

Die Herstellung nahezu aller Einzelteile der Austragvorrichtung 1 geschieht im Kunststoffspritzgussverfahren. Der Mediumspeicher 2 wird insbesondere aus Glas oder Kunststoff hergestellt. Die Schraubenfeder 23 ist in der in Fig. 1 dargestellten Ausführungsform aus Metall hergestellt, kann aber bei entsprechender Umgestaltung auch aus Kunststoff hergestellt sein. Die Mediumröhre 11 ist in der vorliegenden Ausführungsform ebenfalls aus Metall hergestellt, kann aber auch aus Kunststoff oder keramischen Werkstoffen hergestellt werden.

Nach der Produktion der Einzelteile findet eine Vormontage in Baugruppen statt. Das Ziel dieser Vormontage ist es, möglichst viele Montageschritte bereits beim Hersteller der Austragvorrichtung und damit vor der Befüllung des Mediumspeichers 2 mit Medium 17 durchzuführen. Somit wird dem Abfüller des Mediums 17 eine schnelle und kostengünstige Endmontage ermöglicht. Eine erste Baugruppe umfasst das Grundgehäuse 5, in das der Ventilblock 38 und anschließend der mit dem Mediumkanal 11 versehene Pumpenstößel 3 eingesetzt werden. Weiterhin wird in das Grundgehäuse 5 das Wellelement 25 eingesetzt und befestigt. Eine zweite Baugrupe wird aus der Druckhülse 4, der Vorschubhülse 9 und der Schraubenfeder 23 aufgebaut. Nach der Befüllung des Mediumspeichers 2 mit Medium 17 wird das Dichtelemtent 10 eingebracht und somit das Medium 17 dicht gegenüber der Umgebung abgeschlossen. In einem weiteren Montageschritt wird der Mediumspeicher in die Vorschubhülse 9 eingeschoben. Anschließend wird die erste Baugruppe mit dem Grundgehäuse 5 aufgesteckt, wobei der Zentrierkonus 33 der Druckhülse 4 entlang der Einführschräge 22 gleitet und nach Passieren einer Verengung des Aufnahmeraumes 7 die formschlüssige Verbindung zwischen Druckhülse 4 und Grundgehäuse 5 hergestellt wird. Gleichzeitig rastet die Vorschubhülse 9 mit dem Ruherastbereich 36 formschlüssig in die Rastelemente 42 des am Grundgehäuse 5 befestigten Wellelementes 25 ein. Weiterhin wird die Schraubenfeder 23 vorgespannt und hält die Druckhülse 4 in einer Ruheposition.

Um einen Medienaustrag aus der Austragvorrichtung 1 zu bewirken, legt der Benutzer gemäß Fig. 1 vorzugsweise den Mittel- und Zeigefinger auf die Fingerauflage 8, während er gleichzeitig mit dem Daumen einen Druck auf die Daumenauflage 24 ausübt. Dabei muss der Benutzer zumindest eine Betätigungskraft aufbringen, die sich insbesondere aus einer Vorspannung der Schraubenfeder 23 und einer zur Überwindung der formschlüssigen Verbindung zwischen dem Wellelemente 25 und dem Arbeitsbereich 35 der Vorschubhülse 9 notwendigen Deformationskraft sowie einem Reibungsanteil zusammensetzt. Sobald die Betätigungskraft vom Benutzer auf die Austragvorrichtung ausgeübt wird, werden die Rastelemente 19 des Wellelementes 25 durch elastische Deformation der Wellvorsprünge 41 entriegelt und erlauben einen Gleitvorgang des Arbeitsbereiches 35 in Richtung des Applikationsbereiches. Dabei setzen sich sowohl die Druckhülse 4 als auch die Vorschubhülse 9 in Bewegung, wobei der Benutzer nun lediglich noch gegen die Federkraft der Spiralfeder 23 und eine Gleitreibungskraft arbeiten muss.

Sobald ein Druckbund 29 der Vorschubhülse 9 mit einem Mediumspeicherboden 28 in Kontakt tritt, wird dort ein Formschluss zwischen Mediumspeicher 2 und Vorschubhülse 9 ausgebildet, der eine gemeinsame Fortbewegung dieser beiden Bauteile hervorruft. Durch die Bewegung des Mediumspeichers 2 in die Austraghubrichtung A kommt das Dichtelement 10 mit der Schneidspitze 14 der Mediumröhre 11 in Berührung und wird bei zunehmendem Druck des Benutzers auf die Daumenauflage 24 durchstoßen. Dadurch dringt die Mediumröhre 11 in den Medienbehälter 2 ein und das im Medienbehälter 2 eingeschlossene Medium 17 kann zunächst nahezu drucklos in den Mediumkanal 6 einströmen. Eine Druckbeaufschlagung des Mediums 17 findet bei fortschreitender Bewegung des Mediumspeichers 2 in Austraghubrichtung A statt. Die Stößeldruckfläche 15 des Pumpenstößels 3 kommt an der Kolbendruckfläche 16 des Dichtelementes 10 zur Anlage und setzt durch den vom Benutzer aufgebrachten Daumendruck das Medium 17 zunehmend unter Druck. Durch den steigenden Druck im Mediumspeicher 2 fließt das Medium 17 entlang des Mediumkanals 6 in Richtung des Ventilblocks 38, der den Mediumkanal 6 nach oben hin abschließt. Bei Erreichen eines konstruktiv vorgegebenen Mindestdrucks öffnet das aus dem Ventilblock 38 und der Ventilkammer 39 gebildete Ventil. Das Medium 17 wird durch die rapide Querschnittsveränderung in der Austragdüse 40 vernebelt in die Umgebung abgegeben.

Der Austraghub wird beendet, sobald die Druckhülse 4 mit ihrer dem Applikationsbereich zugewandten Stirnseite an einer Stirnseite des Wellelementes 25 anschlägt. Der Arbeitsbereich 35 der Vorschubhülse ist dabei so ausgelegt, dass die Rastelemente 19 des Wellelementes 25 bei Erreichen einer solchen Position in den Funktionsrastbereich 37 formschlüssig eingreifen und die Vorschubhülse in dieser Position festhalten. Da ein weiterer Medienstrom durch das Erreichen einer Blocklänge der Druckhülse 4 verhindert ist, wird der Benutzer den Daumendruck auf die Daumauflage 24 sowie den Vorschubhülsenboden 30 reduzieren. Dadurch führt die in der Schraubenfeder 23 gespeicherte Energie zu einer Bewegung der Druckhülse 4 entgegen der Austraghubrichtung A. Der abschnittsweise geschlitzte Druckkonus 44 gleitet aus dem ursprünglichen Startrastbereich 32 in den Druckrastbereich 31 und liegt mit seinem Anschlagbund 20 wieder an der Anschlagkante 18 der Außenhülle des Aufnahmeraums 7 an. Dadurch wird eine weitere Betätigung der Vorschubhülse 9 durch die Druckhülse 4 ermöglicht, die einen zweiten Austragvorgang bei Aufbringen einer entsprechenden Betätigungskraft zulässt.

Durch die aufgebrachte Betätigungskraft wird in der Anfangsphase des Austraghubes eine innere Spannung in dem Wellelemente 25 hervorgerufen, das bei einer entsprechender Auslegung der Rastelemente 42 zu einer elastischen Deformation der wellenförmigen Wandung des Wellelementes 25 führt, wobei die Rastelemente 41 aus ihrer Position herausgeschwenkt werden können und somit die formschlüssige Verriegelung zwischen dem Wellelement 25 und dem Arbeitsbereich der Vorschubhülse 9 freigeben können.

In einer weiteren nicht dargestellten Ausführungsform sind die Rastelemente 42 an dem Arbeitsbereich 35 der Austragvorrichtung 1 angebracht, während die Ein- oder Ausformungen zur Herstellung eines Formschlusses an den Wellvorsprüngen 41 vorgesehen sind.

## Patentansprüche

1. Austragvorrichtung (1) zur manuellen Erzeugung eines Volumenstroms durch einen Austraghub, mit einem Mediumspeicher (2) für zumindest ein Medium (17), einer Austragöffnung (40), einer Pumpeinrichtung und einem der Pumpeinrichtung zugeordneten Arbeitsbereich (35), der in einer Ruheposition über eine Rasteinrichtung (19, 21, 25, 34) eine lösbare, formschlüssige Verbindung zu einem Haltebereich aufweist, **dadurch gekennzeichnet, dass** die Rasteinrichtung (19, 21, 25, 34) in zumindest einer Ebene orthogonal zu einer Austraghubrichtung (A) einen zumindest abschnittsweise oder umlaufend gewellten Querschnitt aufweist.

2. Austragvorrichtung nach Anspruch 1, **dadurch gekennzeichnet, dass** an mindestens einem dem Arbeitsbereich (35) zugewandten Wellvorsprung (41) der Rasteinrichtung (19, 21, 25, 34) ein Rastelement (42) vorgesehen ist.

3. Austragvorrichtung nach Anspruch 1 oder 2, **dadurch gekennzeichnet, dass** die Rasteinrichtung (19, 21, 25, 34) in dem zumindest abschnittsweise oder umlaufend gewellten Querschnittsbereich durch eine einheitliche oder abschnittsweise variierende Wandstärke zumindest abschnittsweise flexibel gestaltet ist.

4. Austragvorrichtung nach Anspruch 2, **dadurch gekennzeichnet, dass** das Rastelement (42) an einer dem Wellvorsprung (41) abgewandten Stirnfläche eine Kontur aufweist, die der Geometrie des Arbeitsbereichs (35) angepasst ist und die als Führungselement für den Arbeitsbereich (35) vorgesehen ist.

5. Austragvorrichtung nach einem der vorhergehenden Ansprüche, **dadurch gekennzeichnet, dass** der Arbeitsbereich (35) an einer Außenfläche des Mediumspeichers (2) vorgesehen ist.

6. Austragvorrichtung nach einem der vorhergehenden Ansprüche, **dadurch gekennzeichnet, dass** der Arbeitsbereich (35) an einer Außenfläche einer Vorschubhülse (9) vorgesehen ist, wobei die Vorschubhülse (9) den Mediumspeicher (2) zumindest abschnittsweise umgreift, die Vorschubhülse (9) vorzugsweise einen Ruherastbereich (36) und zumindest einen davon beabstandeten Funktionsrastbereich (37) aufweist und begrenzt verschieblich in dem Haltebereich gelagert ist und/oder von einer Druckhülse (4) ansteuerbar angeordnet ist und zumindest eine lösbare, formschlüssige Druckposition zwischen der Vorschubhülse (9) und der Druckhülse (4) vorgesehen ist.

7. Austragvorrichtung nach Anspruch 6, **dadurch gekennzeichnet, dass** die Druckhülse (4) von einem Startrastbereich (32) in zumindest einen weiteren Druckrastbereich (31) entlang der Vorschubhülse (9) überführbar ist.

8. Austragvorrichtung nach einem der Ansprüche 2 bis 7, **dadurch gekennzeichnet, dass** die Rasteinrichtung (19, 21, 25, 34) die Form einer gewellten Hülse hat, wobei im Bereich einer ersten Stirnfläche der Hülse zumindest ein Rastelement (42) und an einer weiteren Stirnfläche eine vorzugsweise als Halteflansch ausgebildete Versteifung vorgesehen ist.

9. Austragvorrichtung nach einem der vorhergehenden Ansprüche, **dadurch gekennzeichnet, dass** die Druckhülse (4), die Vorschubhülse (9), das Rastelement (25) und der Mediumspeicher (2) über insbesondere formschlüssig wirkende Rasteinrichtungen so aufeinander abgestimmt sind, dass zumindest ein zweifacher Austraghub der Austragvorrichtung (1) durchführbar ist.

10. Austragvorrichtung nach Anspruch 1, **dadurch gekennzeichnet, dass** eine den Medienspeicher (2) zumindest abschnittsweise umgreifende Vorschubhülse (9) vorgesehen ist, die für eine formschlüssige Rastverbindung mit der Rasteinrichtung (19, 21, 25, 34) und einer Druckhülse (4) jeweils zumindest zwei voneinander beabstandete Rastbereiche (31, 32, 36, 37) aufweist, und die von der Druckhülse (4) derart ansteuerbar vorgesehen ist, dass durch eine von einem Benutzer mehrfach aufgebrachte Betätigungskraft zumindest ein zweimaliger Austrag des Mediums (17) vornehmbar ist.

## Claims

1. Discharge device (1) for manually producing a volume flow through a discharge stroke, with a medium reservoir (2) for at least one medium (17), a discharge opening (40), a pumping device and a working area (35) associated with said pumping device and which in an inoperative position has a detachable, positive connection with a holding area via a locking device (19, 21, 25, 34), wherein the locking device (19, 21, 25, 34), in at least one plane orthogonal to a discharge stroke direction (A), has an at least zonally or circumferentially corrugated cross-section.

2. Discharge device according to claim 1, wherein on at least one corrugated projection (41) of the locking device (19, 21, 25, 34) facing the working area (35) is provided a locking element (42).

3. Discharge device according to claim 1 or 2, wherein the locking device (19, 21, 25, 34) is at least zonally made flexible by a uniform or sectionally varying wall thickness in the at least zonally or circumferentially corrugated cross-sectional area.

4. Discharge device according to claim 2, wherein, on a face remote from the corrugated projection (41), the locking element (42) has a contour adapted to the geometry of the working area (35) and which is provided as a guide element for said working area (35).

5. Discharge device according to one of the preceding claims, wherein the working area (35) is provided on an outer face of the medium reservoir (2).

6. Discharge device according to one of the preceding claims, wherein the working area (35) is provided on an outer face of a feed sleeve (9), which at least zonally embraces the medium reservoir (2), the feed sleeve (9) preferably having an inoperative locking area (36) and at least one operative locking area (37) spaced therefrom and which is mounted in limited displaceable manner in the holding area and/or is controllable by a pressure sleeve (4) and at least one detachable, positive pressure position is provided between the feed sleeve (9) and the pressure sleeve (4).

7. Discharge device according to claim 6, wherein the pressure sleeve (4) can be transferred along the feed sleeve (9) from a starting locking area (32) into at least one further pressure locking area (31).

8. Discharge device according to one of the claims 2 to 7, wherein the locking device (19, 21, 25, 34) is in the form of a corrugated sleeve and in the vicinity of a first sleeve face is provided at least one locking element (42) and on a further face a stiffening preferably constructed as a holding flange.

9. Discharge device according to one of the preceding claims, wherein the pressure sleeve (4), feed sleeve (9), locking element (25) and medium reservoir (2) are so matched to one another by means of in particular positively acting locking devices that it is possible to at least perform a double discharge stroke of the discharge device (1).

10. Discharge device according to claim 1, wherein a feed sleeve (9) at least zonally embracing the medium reservoir (2) is provided, which has at least two spaced locking areas (31, 32, 36, 37) for a positive locking connection with the locking device (19, 21, 25, 34) and a pressure sleeve (4), and which is controllable by said pressure sleeve (4) in such a way that an actuating force multiply applied by a user permits an at least double discharge of the medium (17).

## Revendications

1. Dispositif de décharge (1) pour produire manuellement un débit volumétrique par une course de décharge, avec un réservoir de produit (2) pour au moins un produit (17), une ouverture de décharge (40), un dispositif de pompage et une zone de travail (35) associée au dispositif de pompage qui présente dans une position de repos par le biais d'un dispositif d'encliquetage (19, 21, 25, 34) une liaison à complémentarité de forme, détachable avec une zone de retenue, **caractérisé en ce que** le dispositif d'encliquetage (19, 21, 25, 34) présente dans au moins un plan orthogonalement à un sens de course de décharge (A) une section ondulée au moins par zones ou de manière périphérique.

2. Dispositif de décharge selon la revendication 1, **caractérisé en ce qu'**un élément d'encliquetage (42) est prévu sur au moins une saillie ondulée (41) dirigée vers la zone de travail (35) du dispositif d'encliquetage (19, 21, 25, 34).

3. Dispositif de décharge selon la revendication 1 ou 2, **caractérisé en ce que** le dispositif d'encliquetage (19, 21, 25, 34) est conçu au moins par zones de manière flexible dans la zone de section ondulée au moins par zones ou de manière périphérique par une épaisseur de paroi uniforme ou variable par zones.

4. Dispositif de décharge selon la revendication 2, **caractérisé en ce que** l'élément d'encliquetage (42) présente sur une face frontale opposée à la saillie ondulée (41) un contour qui est adapté à la géométrie de la zone de travail (35) et qui est prévu comme élément de guidage pour la zone de travail (35).

5. Dispositif de décharge selon l'une quelconque des revendications précédentes, **caractérisé en ce que** la zone de travail (35) est prévue sur une face extérieure du réservoir de produit (2).

6. Dispositif de décharge selon l'une quelconque des revendications précédentes, **caractérisé en ce que** la zone de travail (35) est prévue sur une face extérieure d'une douille d'avance (9), la douille d'avance (9) entourant au moins par zones le réservoir de produit (2), la douille d'avance (9) présentant de préférence une zone d'encliquetage de repos (36) et au moins une zone d'encliquetage fonctionnelle (37) espacée de celle-ci et étant logée à déplacement limité dans la zone de retenue et/ou étant disposée de manière à pouvoir être commandée par une douille de pression (4) et au moins une position de pression à complémentarité de forme, détachable entre la douille d'avance (9) et la douille de pression (4) étant prévue.

7. Dispositif de décharge selon la revendication 6, **caractérisé en ce que** la douille de pression (4) peut être transférée d'une zone d'encliquetage de départ (32) dans au moins une autre zone d'encliquetage de pression (31) le long de la douille d'avance (9).

8. Dispositif de décharge selon l'une quelconque des revendications 2 à 7, **caractérisé en ce que** le dispositif d'encliquetage (19, 21, 25, 34) présente la forme d'une douille ondulée, dans la zone d'une première face frontale de la douille, au moins un élément d'encliquetage (42) et sur une autre face frontale, un renforcement réalisé de préférence comme une bride de retenue étant prévus.

9. Dispositif de décharge selon l'une quelconque des revendications précédentes, **caractérisé en ce que** la douille de pression (4), la douille d'avance (9), l'élément d'encliquetage (25) et le réservoir de produit (2) sont ajustés les uns sur les autres par des dispositifs d'encliquetage agissant notamment par complémentarité de forme de telle sorte qu'au moins une double course de décharge du dispositif de décharge (1) puisse être réalisée.

10. Dispositif de décharge selon la revendication 1, **caractérisé en ce qu'**une douille d'avance (9) entourant au moins par zones le réservoir de produit (2) est prévue, laquelle présente pour une liaison par encliquetage à complémentarité de forme avec le dispositif d'encliquetage (19, 21, 25, 34) et une douille de pression (4) respectivement au moins deux zones d'encliquetage (31, 32, 36, 37) espacées l'une de l'autre, et qui est prévue de manière à pouvoir être commandée par la douille de pression (4) de telle sorte qu'au moins une double décharge du produit (17) puisse être entreprise par une force d'actionnement appliquée de manière répétée par un utilisateur.
